(19) Europäisches Patentamt
European Patent Office
Office européen des brevets

(11) **EP 2 550 946 B1**

(12) **EUROPEAN PATENT SPECIFICATION**

(45) Date of publication and mention
of the grant of the patent:
**29.10.2014 Bulletin 2014/44**

(51) Int Cl.:
***A61F 13/49*** *(2006.01)*  ***A61F 13/53*** *(2006.01)*
***A61F 13/533*** *(2006.01)*  ***A61F 13/15*** *(2006.01)*

(21) Application number: **10848390.0**

(22) Date of filing: **25.03.2010**

(86) International application number:
**PCT/JP2010/055222**

(87) International publication number:
**WO 2011/117997 (29.09.2011 Gazette 2011/39)**

(54) **WATER-ABSORBING SHEET STRUCTURE**

WASSERABSORBIERENDE FOLIENSTRUKTUR

STRUCTURE EN FEUILLETS ABSORBANT L'EAU

(84) Designated Contracting States:
**AT BE BG CH CY CZ DE DK EE ES FI FR GB GR
HR HU IE IS IT LI LT LU LV MC MK MT NL NO PL
PT RO SE SI SK SM TR**

(43) Date of publication of application:
**30.01.2013 Bulletin 2013/05**

(73) Proprietor: **Sumitomo Seika Chemicals Co., Ltd.
Hyogo 675-0145 (JP)**

(72) Inventors:
• **TAKATORI, Junichi
Hyogo 672-8076 (JP)**
• **MATSUSHITA, Hideki
Hyogo 672-8076 (JP)**

• **SAKATA, Jun
Hyogo 672-8076 (JP)**
• **INABA, Haruka
Hyogo 672-8076 (JP)**

(74) Representative: **Jackson, Martin Peter et al
J A Kemp
14 South Square
Gray's Inn
London WC1R 5JJ (GB)**

(56) References cited:
JP-A- 2 053 968     JP-A- 2003 510 165
JP-A- 2006 051 218   US-A- 5 451 442
US-A1- 2003 028 165  US-A1- 2003 139 719
US-A1- 2003 187 417  US-B1- 7 195 810

**Description**

TECHNICAL FIELD

The present invention relates to a water-absorbent sheet structure which can be used in the fields of hygienic materials and the like. More specifically, the present invention relates to a water-absorbent sheet structure which is thin and can be suitably used in absorbent articles, such as disposable diapers and incontinence pads. In addition, the present invention relates to an absorbent article such as disposable diapers and incontinence pads, using the water-absorbent sheet structure.

BACKGROUND ART

Absorbent articles represented by disposable diapers or the like have a structure in which an absorbent material for absorbing a liquid such as a body liquid is sandwiched with a flexible liquid-permeable surface sheet (top sheet) positioned on a side contacting a body and a liquid-impermeable backside sheet (back sheet) positioned on a side opposite to that contacting the body.

Conventionally, there have been increasing demands for thinning and light-weighing of absorbent articles, from the viewpoint of designing property, convenience upon carrying, and efficiency upon distribution. Further, in the recent years, there have been growing needs for so-called eco-friendly intentions, in which resources are effectively utilized so that use of natural materials that require a long time to grow such as trees is avoided as much as possible, from the viewpoint of environmental protection.

In view of the above, as a water-absorbent sheet structure containing a very small amount of crushed pulp fibers of wood or the like, having excellent fundamental properties, such as fast liquid permeation rate, sufficient liquid absorbent properties, small amount of liquid re-wet, small liquid leakage, and shape retaining ability, to accomplish thinning, a water-absorbent sheet structure having a structure comprising a given amount of a water-absorbent resin and a given amount of a hot melt adhesive sandwiched with two or more sheets of hydrophilic nonwoven fabrics having a given basis weight has been proposed (see, for example, Patent Publication 1).

On the other hand, it has been proposed that the conventional absorbent materials mentioned above are subjected to embossing, in order to improve absorption rate, fittability, and an effect of preventing shape deformation (see, for example, Patent Publication 2).

PRIOR ART PUBLICATIONS

PATENT PUBLICATIONS

Patent Publication 1: WO 2010/004894
Patent Publication 2: Japanese Patent Laid-Open No. Hei-5-300922

US 2003/0028165 discloses a laminate web comprising a first web, a second web joined to the first web at a plurality of discrete bond sites; and a third material disposed between at least a portion of the first and second nonwovens. The third material is aperture in regions adjacent the bond sites, such that the first and second nonwoven webs are joined through the apertures. In one embodiment an aperture laminate web is disclosed, having a first extensible web having a first elongation to break, and a second extensible web joined to the first extensible web at a plurality of bond sites, the second extensible web having elongation to break. A third web material is disposed between the first and second nonwovens, the third web material having a third elongation to break which is less than both of the first or second elongations to break. In a further embodiment, an aperture laminate web is disclosed, having first and second extensible webs being joined at a plurality of discrete bond sites and a third material disposed between the first and second nonwoven webs. The first and second nonwoven webs are in fluid communication via the apertures and have distinct regions being differentiated by at least one property selected from the group consisting of basis weight, fiber orientation, thickness, and density.

SUMMARY OF THE INVENTION

PROBLEMS TO BE SOLVED BY THE INVENTION

While the water-absorbent sheet structure disclosed in Patent Publication 1 is sufficiently excellent in the fundamental properties mentioned above, a proposal of a water-absorbent sheet structure having even more excellent properties, especially in fast liquid permeation rate, small liquid leakage, and shape retaining ability has been earnestly desired.

The water-absorbent sheet structure mentioned above has been tried to be subjected to embossing disclosed in Patent Publication 2 or the like; however, when the water-absorbent sheet structure absorbs a liquid, it has been found that the embossing mentioned above inhibits a water-absorbent resin in an absorbent layer from absorbing water to allow swelling, so that the liquid absorbent properties of the water-absorbent sheet structure are worsened. Also, it has been found that the embossing would be lost by swelling of the water-absorbent resin mentioned above, so that in some cases, the embossing may not effectively act against the subsequent liquid absorption.

An object of the present invention is to provide a water-absorbent sheet structure having excellent liquid permeability, small liquid leakage, and excellent shape retaining ability, and is capable of accomplishing thinning.

MEANS TO SOLVE THE PROBLEMS

The present invention provides a water-absorbent sheet structure comprising a structure according to claim 1.

EFFECTS OF THE INVENTION

The water-absorbent sheet structure of the present invention exhibits some excellent effects that a water-absorbent sheet structure has excellent shape retaining ability even when the structure is thin, so that the water absorbent sheet structure does not undergo deformation of the form before liquid absorption or after the absorption thereof, and is capable of sufficiently exhibiting absorbent properties such as excellent liquid permeability and small liquid leakage. Therefore, the water-absorbent sheet structure according to the present invention is used for an absorbent material such as disposable diapers, whereby hygienic materials which are thin and have excellent design property of external appearance, and at the same time not having disadvantages such as liquid leakage can be provided. Also, the water-absorbent sheet structure according to the present invention can be used in agricultural fields and fields of construction materials other than the field of hygienic materials.

BRIEF DESCRIPTION OF THE DRAWINGS

[Figure 1] A cross-sectional view schematically showing one embodiment of a water-absorbent sheet structure according to the present invention before absorption of saline solution (dry state).
[Figure 2] A cross-sectional view schematically showing one embodiment of a water-absorbent sheet structure according to the present invention after absorption of saline solution.
[Figure 3] One embodiment of embossing patterns usable in the present invention.
[Figure 4] Another embodiment of embossing patterns usable in the present invention.
[Figure 5] Another embodiment of embossing patterns usable in the present invention.
[Figure 6] Another embodiment of embossing patterns usable in the present invention.
[Figure 7] Another embodiment of embossing patterns usable in the present invention.
[Figure 8] A schematic view showing an outline of the constitution of an apparatus used for carrying out a slope leakage test for a water-absorbent sheet structure.

MODES FOR CARRYING OUT THE INVENTION

The water-absorbent sheet structure according to the present invention is a water-absorbent sheet structure comprising a structure in which an absorbent layer containing a water-absorbent resin is sandwiched with hydrophilic nonwoven fabrics from an upper side and a lower side of the absorbent layer, in which at least one side of the topside and the underside of the water-absorbent sheet structure is subjected to embossing. By satisfying specified thickness conditions and specified embossing retaining conditions in the water-absorbent sheet structure, a thin water-absorbent sheet structure having fast liquid permeation rate, small liquid leakage, and excellent shape retaining ability can be realized.

The water-absorbent sheet structure according to the present invention may be in an embodiment where a hydrophilic fiber such as pulp fiber is admixed between the hydrophilic nonwoven fabrics together with the water-absorbent resin in an amount that would not impair the effects of the present invention. However, it is preferable that the structure is in an embodiment where a hydrophilic fiber is substantially not contained, from the viewpoint of thinning.

As the water-absorbent resins usable in the water-absorbent sheet structure according to the present invention, known water-absorbent resins can be used. For example, the water-absorbent resin includes hydrolysates of starch-acrylonitrile graft copolymers, neutralized products of starch-acrylic acid graft polymers, saponified products of vinyl acetate-acrylic acid ester copolymers, partially neutralized products of polyacrylic acid, and the like. Among these water-absorbent resins, the partially neutralized products of polyacrylic acids are preferably used, from the viewpoint of production amount, production costs, water-absorbent properties, and the like. Methods for synthesizing partially neutralized products of polyacrylic acid include reversed phase suspension polymerization method, aqueous solution polymerization method, and the like. Among these polymerization methods, the water-absorbent resins obtained according to reversed phase suspension polymerization method are preferably used, from the viewpoint of excellent flowability of the resulting particles, smaller amounts of fine powder, high water-absorbent properties, such as liquid absorption capacity (expressed by indices such as water-retention capacity, effective amount of water absorbed, water-absorption capacity under load), and water-absorption rate.

The above-mentioned partially neutralized product of a polyacrylic acid has a degree of neutralization of preferably 50% by mol or more, and more preferably from 70 to 90% by mol, from the viewpoint of increasing an osmotic pressure of the water-absorbent resin, thereby increasing water-absorbent properties.

The water-absorbent resin is contained in the water-absorbent sheet structure according to the present invention of preferably from 100 to 1,000 g per 1 $m^2$ of the water-absorbent sheet structure, i.e. 100 to 1,000 $g/m^2$, more preferably from 150 to 800 $g/m^2$, even more preferably from 200 to 700 $g/m^2$, and still even more preferably from 220 to 600 $g/m^2$, from the viewpoint of obtaining sufficient liquid absorbent properties, when the above-mentioned water-absorbent sheet structure is used for an absorbent article. It is preferable that the water-absorbent resin is contained in an amount of 100 $g/m^2$ or more, from the viewpoint of exhibiting sufficient liquid absorbent properties as a water-absorbent sheet structure, thereby suppressing re-wetting, and it is preferable that the water-absorbent resin is contained in an amount of 1,000 $g/m^2$ or less, from the viewpoint of suppressing the gel blocking phenomenon from being caused, exhibiting liquid diffusibility as a water-absorbent sheet structure, and further improving a liquid permeation rate.

The hydrophilic nonwoven fabrics usable in the water-absorbent sheet structure according to the present invention are not particularly limited, as long as the hydrophilic nonwoven fabrics are known hydrophilic nonwoven fabrics in the field of art. The hydrophilic nonwoven fabrics include hydrophilic nonwoven fabrics made of polyolefin fibers such as polyethylene (PE) and polypropylene (PP); polyester fibers such as polyethylene terephthalate (PET), polytrimethylene terephthalate (PTT), and polyethylene naphthalate (PEN); polyamide fibers such as nylon; rayon fibers, and other synthetic fibers; hydrophilic nonwoven fabrics produced by mixing cotton, silk, hemp, pulp (cellulose) fibers, or the like, from the viewpoint of liquid permeability, flexibility and shape retaining ability upon forming into the above-mentioned water-absorbent sheet structure. Among these hydrophilic nonwoven fabrics, the hydrophilic nonwoven fabrics made of synthetic fibers are preferably used, from the viewpoint of increasing the shape retaining ability of the water-absorbent sheet structure. Especially, hydrophilic nonwoven fabrics made of rayon fibers, polyolefin fibers, and polyester fibers are preferred. In addition, the above-mentioned hydrophilic nonwoven fabrics made of synthetic fibers may contain a small amount of pulp fibers to an extent that would not increase the thickness of the water-absorbent sheet structure obtained. These hydrophilic nonwoven fabrics may be hydrophilic nonwoven fabrics made of single fibers mentioned above, or hydrophilic nonwoven fabrics made of two or more kinds of fibers used in combination.

More specifically, spunbond nonwoven fabrics made of fibers selected from the group consisting of polyolefin fibers, polyester fibers and blends thereof are more preferred, from the viewpoint of increasing shape retaining ability of the water-absorbent sheet structure, and preventing pass of the water-absorbent resin through the nonwoven fabric. In addition, spunlace nonwoven fabrics made of rayon fibers as a main component are also more preferred as the nonwoven fabrics used in the present invention, from the viewpoint of even more increasing liquid absorbent properties and flexibility upon formation of the water-absorbent sheet structure. Among the spunbond nonwoven fabrics mentioned above, spunbond-meltblown-spunbond (SMS) nonwoven fabrics and spunbond-meltblown-meltblown-spunbond (SMMS) nonwoven fabrics, which have a multi-layered structure of polyolefin fibers are more preferably used, and the SMS nonwoven fabrics and the SMMS nonwoven fabrics each made of polypropylene fibers as a main component are especially preferably used. On the other hand, as the above-mentioned spunlace nonwoven fabrics, those of proper blends of main component rayon fibers with polyolefin fibers and/or polyester fibers are preferably used, and among them, rayon-PET nonwoven fabrics and rayon-PET-PE nonwoven fabrics are preferably used. The above-mentioned nonwoven fabrics may contain a small amount of pulp fibers to an extent that would not increase the thickness of the water-absorbent sheet structure.

The hydrophilic nonwoven fabric mentioned above is preferably a hydrophilic nonwoven fabric having an appropriate bulkiness and a large basis weight, from the viewpoint of giving the water-absorbent sheet structure according to the present invention excellent liquid permeability, flexibility, shape retaining ability and cushioning property, and speeding up the liquid permeation rate of the water-absorbent sheet structure. The hydrophilic nonwoven fabric has a basis weight of preferably from 5 to 300 $g/m^2$, more preferably from 10 to 200 $g/m^2$, even more preferably from 11 to 100 $g/m^2$, and still even more preferably from 13 to 50 $g/m^2$. Also, the hydrophilic nonwoven fabric has a thickness of preferably in the range of from 200 to 1,500 $\mu$m, more preferably in the range of from 250 to 1,200 $\mu$m, and even more

preferably in the range of from 300 to 1,000 μm.

In the water-absorbent sheet structure according to present invention, it is preferable that the absorbent layer further contains an adhesive, from the viewpoint of increasing the shape retaining ability of the water-absorbent sheet structure obtained. In a case where an adhesive is used, the adhesive includes, for example, rubber-based adhesives such as natural rubbers, butyl rubbers, and polyisoprene; styrene-based elastomer adhesives such as styrene-isoprene block copolymers (SIS), styrene-butadiene block copolymers (SBS), styrene-isobutylene block copolymers (SIBS), and styrene-ethylene-butylene-styrene block copolymers (SEBS); ethylene-vinyl acetate copolymer (EVA) adhesives; ethylene-acrylic acid derivative copolymer-based adhesives such as ethylene-ethyl acrylate copolymer (EEA), and ethylene-butyl acrylate copolymer (EBA); ethylene-acrylic acid copolymer (EAA) adhesives; polyamide-based adhesives such as copolymer nylons and dimer acid-based polyamides; polyolefin-based adhesives such as polyethylenes, polypropylenes, atactic polypropylenes, and copolymeric polyolefins; polyester-based adhesives such as polyethylene terephthalate (PET), polybutylene terephthalate (PBT), and copolymeric polyesters; and acrylic-based adhesives. Among these adhesives, the ethylene-vinyl acetate copolymer adhesives, the styrene-based elastomer adhesives, the polyolefin-based adhesives, and the polyester-based adhesives are preferably used, from the viewpoint of high adhesive strength, thereby making it possible to prevent exfoliation of a hydrophilic nonwoven fabric and scattering of the water-absorbent resin in the water-absorbent sheet structure. These adhesives may be used alone, or they may be used in combination of two or more kinds.

The above-mentioned adhesive has a melting temperature or softening temperature of preferably from 60° to 180°C, and more preferably from 70° to 150°C, from the viewpoint of sufficiently fixing a water-absorbent resin to a hydrophilic nonwoven fabric, and at the same time preventing thermal deterioration or deformation of the hydrophilic nonwoven fabric.

In addition, the holding strength of the above-mentioned adhesive cannot be unconditionally determined because the holding strength differs depending upon the hydrophilic nonwoven fabrics and the like used. The adhesive has a holding strength of preferably 1,000 minutes or longer, more preferably 1,100 minutes or longer, and even more preferably 1,200 minutes or longer, from the viewpoint of the shape retaining ability of the embossing and the effect sustainability of the embossing when the water-absorbent sheet structure absorbs a liquid. The holding strength of the adhesive as used herein is a value obtainable by a measurement method set forth below.

The adhesive in the water-absorbent sheet structure according to the present invention is contained in an amount preferably in the range of from 0.05 to 2.0 times, more preferably in the range of from 0.08 to 1.5 times, and even more preferably in the range of from 0.1 to 1.0 time the amount of the above-mentioned water-absorbent resin contained (mass basis). It is preferable that the adhesive is contained in an amount of 0.05 times or more, from the viewpoint of having sufficient adhesion, thereby preventing exfoliation of the hydrophilic nonwoven fabrics themselves or scattering of the water-absorbent resin, increasing shape retaining ability of a water-absorbent sheet structure, and further increasing shape retaining ability of the embossing and effect sustainability of the embossing. It is preferable that the adhesive is contained in an amount of 2.0 times or less, from the viewpoint of avoiding the inhibition of the swelling of the water-absorbent resin due to too strong adhesion to each other, thereby improving a liquid permeation rate or liquid leakage of a water-absorbent sheet structure.

In the water-absorbent sheet structure according to the present invention, the absorbent layer formed between the hydrophilic nonwoven fabrics contains at least a water-absorbent resin, and the water-absorbent sheet structure is formed by, for example, evenly dispersing a mixed powder of a water-absorbent resin and an adhesive on a hydrophilic nonwoven fabric, further overlaying with a hydrophilic nonwoven fabric, and subjecting overlaid layers to heating, if necessary, heating under pressure, near a melting temperature of the adhesive. Alternatively, the water-absorbent sheet structure is also formed by evenly dispersing a water-absorbent resin over an adhesive-coated, hydrophilic nonwoven fabric, further overlaying with an adhesive-coated hydrophilic nonwoven fabric, and subjecting overlaid layers to heating, if necessary, under pressure, or by sandwiching a water-absorbent resin between hydrophilic nonwoven fabrics, and thereafter subjecting overlaid layers to thermal embossing or the like.

The water-absorbent sheet structure according to the present invention can be produced by, for example, a method as described hereinbelow.

(a) A mixed powder of a water-absorbent resin and an adhesive is evenly dispersed over a hydrophilic nonwoven fabric, a hydrophilic nonwoven fabric is further overlaid thereto, and the overlaid layers are subjected to pressing while heating near a melting temperature of the adhesive.

(b) A mixed powder of a water-absorbent resin and an adhesive is evenly dispersed over a hydrophilic nonwoven fabric, and passed through a heating furnace to fix the powder to an extent that the powder does not scatter. A hydrophilic nonwoven fabric is overlaid thereto, and the overlaid layers are subjected to pressing while heating.

(c) An adhesive is melt-coated over a hydrophilic nonwoven fabric, a water-absorbent resin is immediately thereafter evenly dispersed thereto to form a layer, and further a hydrophilic nonwoven fabric to which an adhesive is melt-coated is overlaid from an upper side in a manner that a coated side of the adhesive is facing the side of the dispersed water-absorbent resin, and the overlaid layers are subjected to pressing, or pressing, if necessary, with heating, using a roller press or the like.

(d) A water-absorbent resin is evenly dispersed over a hydrophilic nonwoven fabric, a hydrophilic nonwoven fabric is further overlaid thereto, and the overlaid layers are subjected to thermal embossing, thereby subjecting the hydrophilic nonwoven fabrics themselves to pressing while heating.

A water-absorbent sheet structure having a structure that an absorbent layer containing a water-absorbent resin is sandwiched with two sheets of hydrophilic nonwoven fabrics from an upper side and a lower side of the absorbent layer can be obtained by, for example, producing a water-absorbent sheet structure according to the method shown in any one of these (a) to (d). Among them, the methods of (a), (c) and (d) are more preferred, from the viewpoint of convenience in the production method and high production efficiency. Here, the water-absorbent sheet structure can also be produced by combining the methods exemplified in (a) to (d). The number of sheets of the hydrophilic nonwoven fabrics are preferably 2 sheets or more, and more preferably 2 sheets.

In addition, the water-absorbent sheet structure according to the present invention may properly be formulated with an additive such as a deodorant, an anti-bacterial agent, or a gel stabilizer.

In the water-absorbent sheet structure according to the present invention, at least one side of the topside and the underside of the above-mentioned water-absorbent sheet structure is subjected to embossing, and both the sides may be subjected to embossing.

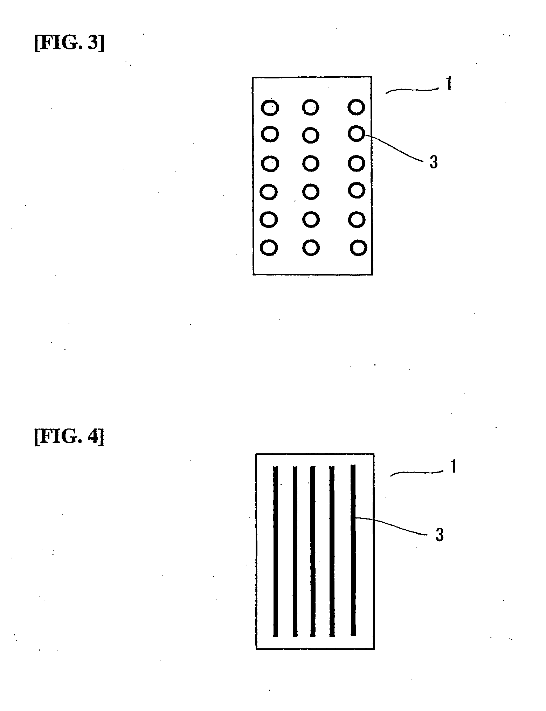

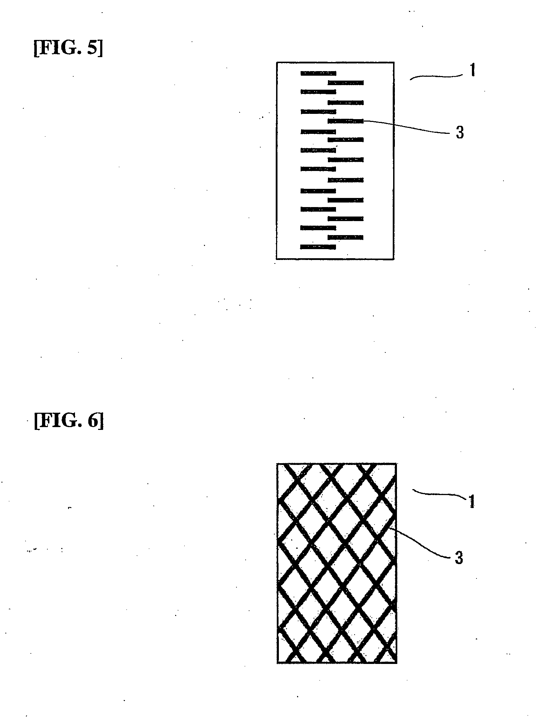

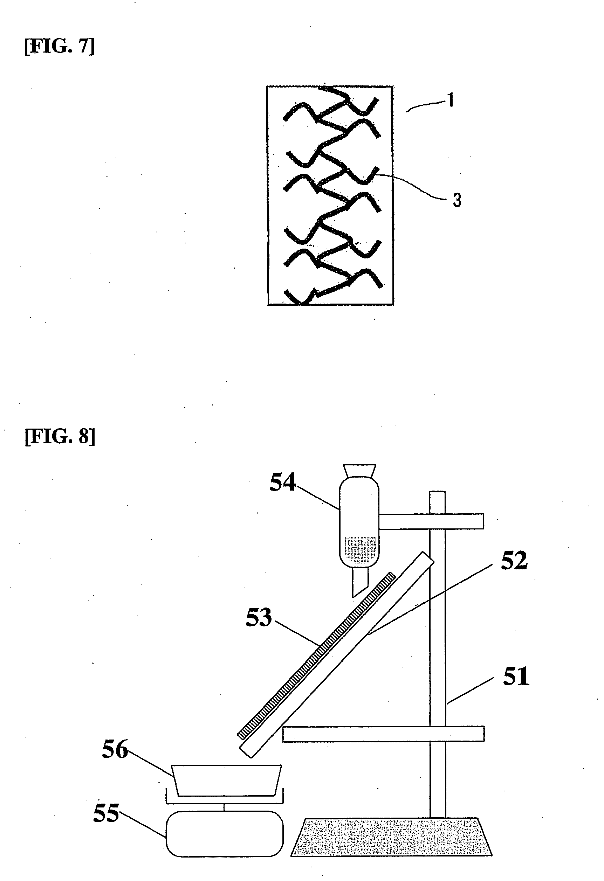

The embossing patterns subjected to the water-absorbent sheet structure according of present invention are not particularly limited, and the embossing patterns include patterns of dotted forms (see, for example, Figure 3), straight lines (see, for example, Figures 4 and 5), curves, wavy forms, and patterns of a combination thereof (see, for example, Figures 6 and 7), and the like. Among these patterns, the patterns of Figures 6 and 7 are preferably used, from the viewpoint of allowing the resulting water-absorbent sheet structure to accomplish a fast liquid permeation rate and a small amount of liquid leakage.

The embossing subjected to the water-absorbent sheet structure according to the present invention has an areal percentage preferably within the range of from 3 to 25%, more preferably within the range of from 4 to 20%, and even more preferably within the range of from 5 to 15%, of the area of the side subjected to embossing of the water-absorbent sheet structure. The areal percentage of embossing is preferably 3% or more, from the viewpoint of accelerating the liquid diffusion from the embossing part and speeding up a permeation rate of a liquid, and from the viewpoint of preventing deformation of the form of the water-absorbent sheet structure due to fixation of the water-absorbent resin to the water-absorbent sheet structure. The areal percentage of embossing is preferably 25% or less, from the viewpoint of preventing the liquid from being diffused which could take place before allowing it to be absorbed to the water-absorbent sheet structure, and preventing a liquid leakage from a water-absorbent sheet structure, from the viewpoint of not inhibiting the swelling of the water-absorbent resin, and from the viewpoint of softening a feel of the water-absorbent sheet structure obtained.

In the water-absorbent sheet structure according to the present invention, a method of subjecting the water-absorbent sheet structure to embossing includes a method using a pressure, heat, ultrasonic wave or an adhesive, and the like. In addition, a method of a combination thereof may be also used. Here, when subjected to embossing, the water-absorbent sheet structure may be directly subjected to embossing during pressing in the method for production mentioned above, or a water-absorbent sheet structure before being subjected to embossing is first produced, and the water-absorbent sheet structure may then be separately subjected to embossing.

The water-absorbent sheet structure according to the present invention has one feature in that when the water-absorbent sheet structure is allowed to absorb the saline solution in an amount of 4 L per 1 m$^2$ of the water-absorbent sheet structure (4 L/m$^2$), the water-absorbent sheet structure satisfies both the following relationships of (A) and (B):

(A) an expansion thickness ratio, i.e. T2/T1, of 2 or more, and
(B) an expansion embossing depth, i.e. (T2 - t2)/T2, of 0.7 or more,

wherein T1 is a thickness (mm) of the water-absorbent sheet structure before absorption of the saline solution; T2 is a thickness (mm) of the water-absorbent sheet structure after absorption of the saline solution; and t2 is an embossing thickness (mm) in the water-absorbent sheet structure after absorption of the saline solution, which are values evaluated by a measurement method set forth below.

The above-mentioned expansion thickness ratio is an index showing a degree of swellability of the water-absorbent resin in the surroundings of the embossing when the water-absorbent sheet structure absorbs a liquid. The expansion thickness ratio is 2 or more, preferably from 3 to 20, and more preferably from 4 to 15. In a case where an expansion thickness ratio is less than 2, when the water-absorbent resin in the absorbent layer absorbs a liquid to allow swelling, the absorbent layer would be in a state of being pressed by upper and lower sides of the hydrophilic nonwoven fabrics, so that the swelling of the water-absorbent resin is inhibited, thereby lowering the absorbent abilities of the water-absorbent sheet structure, whereby making it likely to cause a liquid leakage.

The above-mentioned expansion embossing depth is an index showing a degree of shape retaining ability of the embossing when the water-absorbent sheet structure absorbs a liquid. The expansion embossing depth is 0.7 or more, preferably 0.8 or more, and more preferably 0.9 or more. In a case where an expansion embossing depth is less than 0.7, when the water-absorbent resin in the absorbent layer absorbs a liquid to allow swelling, the shape of embossing is lost, so that the embossing does not effectively act upon the subsequent liquid absorption. Specifically, since the shape of embossing is lost, the liquid permeation rate in the water-absorbent sheet structure is slowed down, or the water-absorbent sheet structure undergoes deformation of the form.

A method of setting T1, T2 and t2 mentioned above to desired levels includes methods for proper adjustments of an amount of the water-absorbent resin, an amount of the adhesive in the absorbent layer, and an areal percentage of embossing subjected to the water-absorbent sheet structure. More specifically, T1 can be set to a desired level by, for example, adjusting a thickness of the hydrophilic nonwoven fabrics and an amount of the water-absorbent resin. T2 can be increased in its value by, for example, reducing an areal percentage of embossing, or increasing an amount of the water-absorbent resin. Further, t2 can be lowered in its value by, for example, increasing an amount of the adhesive, or using an adhesive having an even larger holding strength.

In the present invention, the above-mentioned water-absorbent sheet structure can also take a structure in which a part or entire side of the absorbent layer thereof is fractionated into an upper side primary absorbent layer and a lower side secondary absorbent layer by using an appropriate breathable fractionating layer in a perpendicular direction (the thickness direction of the sheet structure). By having the above structure, the liquid absorbent properties of the water-absorbent sheet structure, especially slope liquid leakage, are dramatically improved.

The above-mentioned breathable fractionating layer has appropriate breathability and liquid-permeability, which may be a layer in which a particle-form substance such as a water-absorbent resin does not substantially pass therethrough. Specific examples thereof include reticular products such as nets having fine pores made of PE or PP fibers; porous films such as perforated films; sanitary papers such as tissue paper; and cellulose-containing synthetic fiber nonwoven fabrics such as air laid nonwoven fabrics made of pulp/PE/PP, or synthetic fiber nonwoven fabrics made of rayon fibers, polyolefin fibers, and polyester fibers, and the like. Among them, the same nonwoven fabrics as those used in sandwiching the absorbent layer in the present invention are preferably used, from the viewpoint of the properties of the water-absorbent sheet structure obtained.

The water-absorbent resin in the secondary absorbent layer is used in an amount of preferably in the range of from 0.01 to 1.0 time, more preferably in the range of from 0.05 to 0.8 times, and even more preferably in the range of from 0.1 to 0.5 times the amount of the water-absorbent resin used of the primary absorbent layer (mass ratio). The water-absorbent resin in the secondary absorbent layer is preferably 0.01 times or more, from the viewpoint of sufficiently exhibiting liquid absorbent properties of the secondary absorbent layer, and preventing liquid leakage, and the water-absorbent resin is preferably 1.0 time or less, from the viewpoint of increasing dry feel at the surface after the liquid absorption and reducing amount of re-wet.

The liquid absorbent properties of the water-absorbent sheet structure according to the present invention are influenced by the water-absorbent properties of the water-absorbent resin used. Therefore, it is preferable that the water-absorbent resin of the primary absorbent layer to be used in the present invention is those selected with favorable ranges in absorbent properties, by taking the constitution of each component of the water-absorbent sheet structure or the like into consideration. In addition, the water-absorbent resin of the secondary absorbent layer may be identical to or different from the water-absorbent resin of the primary absorbent layer.

More specifically, an embodiment where a water-absorbent resin used in at least one of the absorbent layers is a water-absorbent resin obtained by reversed phase suspension polymerization method is preferred, an embodiment where a water-absorbent resin used in a secondary absorbent layer is a water-absorbent resin obtained by reversed phase suspension polymerization method is more preferred, and an embodiment where both the water-absorbent resins used in the primary absorbent layer and the secondary absorbent layer are water-absorbent resins obtained by reversed phase suspension polymerization method is even more preferred.

The water-absorbent sheet structure according to the present invention has one feature in the viewpoint of enabling thinning of the sheet. When the use in absorbent articles, such as disposable diapers, is taken into consideration, the water-absorbent sheet structure has a thickness, in a dry state, of preferably 4 mm or less, more preferably 3 mm or less, and even more preferably from 0.5 to 2 mm. The dry state refers to a state before which the water-absorbent sheet structure absorbs a liquid. In the present specification, the thickness of the water-absorbent sheet structure in a dry state is a value evaluated by a measurement method set forth below.

The water-absorbent sheet structure according to the present invention has one feature that the sheet structure has a fast liquid permeation rate, and the water-absorbent sheet structure has a total permeation rate of preferably 50 seconds or less, and more preferably 48 seconds or less, when its use in an absorbent article is taken into consideration. In the present specification, the total permeation rate of the water-absorbent sheet structure is a value obtainable by a measurement method set forth below.

Further, the water-absorbent sheet structure according to the present invention has one feature that the sheet structure has smaller slope liquid leakage, and the water-absorbent sheet structure has a leakage index of preferably 150 or less, and more preferably 100 or less, when its use in an absorbent article is taken into consideration. In the present specification, the leakage index of the water-absorbent sheet structure is a value obtainable by a measurement method set forth below.

Further, the water-absorbent sheet structure according to the present invention has one feature that the sheet structure has a small amount of re-wet after the liquid permeation. The amount of re-wet of the liquid in the water-absorbent sheet structure is preferably 12 g or less, and more preferably 10 g or less, when its use in an absorbent article is taken into consideration. In the present specification, the amount of re-wet of the liquid in the water-absorbent sheet structure is a value obtainable by a measurement method set forth below.

An absorbent article according to the present invention can be obtained by sandwiching a water-absorbent sheet structure according to the present invention between a liquid-permeable sheet and a liquid-impermeable sheet. In a case where a side subjected to embossing is one side of the water-absorbent sheet structure, it is preferable that a liquid-permeable sheet is placed on a side subjected to embossing. As the liquid-permeable sheet and the liquid-impermeable sheet mentioned above, known sheets used in the field of the present invention can be used, and as to a method of sandwiching with these sheets, known methods can be employed.

EXAMPLES

The present invention will be specifically described hereinbelow by the Examples, without intending to limit the scope of the present invention thereto.

The measurements defined in the present specification were made and evaluated in accordance with the following methods.

[Holding Strength of Adhesive]

A polyethylene film (length: 150 mm, and width: 25 mm) was evenly coated with an adhesive so as to have a thickness of 50 $\mu$m, to provide a test piece. The test piece was adhered to a stainless steel plate (length: 125 mm, and width: 50 mm) in a manner so that an area of the test piece having the dimensions of length 25 mm × width 25 mm contacted one end of the stainless steel plate, and a part of the test piece which was not adhered was folded over with its adhered side in the inner side. The test piece was pressed from a top thereof by moving a 2 kg elastic roller backward and forward once at a rate of 5 mm/second.

After 20 minutes, one side of the stainless steel plate was held so that the stainless steel plate and the test piece were hung vertically, and a 1 kg weight was attached to an end of the folded-over part of the test piece.

A time period from which the test piece was removed to a point the test piece fell off from the stainless steel plate was measured. The measurements were conducted for 3 sheets of test pieces, and an average thereof was defined as holding strength of the adhesive. Here, when the time exceeded 1,440 minutes (24 hours), the holding strength was evaluated as "1,440 minutes or longer."

[Thickness of Water-Absorbent Sheet Structure in Dry State]

The water-absorbent sheet structure obtained was directly used as a sample (10 cm × 30 cm). Here, in a case where a water-absorbent sheet structure obtained did not have dimensions of 10 cm × 30 cm, the water-absorbent sheet structure was cut into rectangular strips having dimensions of 10 cm × 30 cm in a manner that a longitudinal direction thereof is to be in a length direction (machine feeding direction) of the nonwoven fabric, and used as a sample.

The thickness was measured using a thickness measurement instrument (manufactured by Kabushiki Kaisha Ozaki Seisakusho, model number: J-B) at three measurement sites taken in a longitudinal direction, on the left end, the center, and the right end; for example, the left end was set at a site 3 cm away from the left side, the center was set at a site 15 cm away therefrom, and the right end was set at a site 27 cm away therefrom. The measurement value for thickness was obtained by measuring three times at each site, and an average for each site was obtained. Further, the values at the left end, the center, and the right end were averaged, which was defined as a thickness of an overall water-absorbent sheet structure.

[Expansion Thickness Ratio and Expansion Embossing Depth of Water-Absorbent Sheet Structure]

A water-absorbent sheet structure was cut into the dimensions of 5 cm × 5 cm and used as a sample.

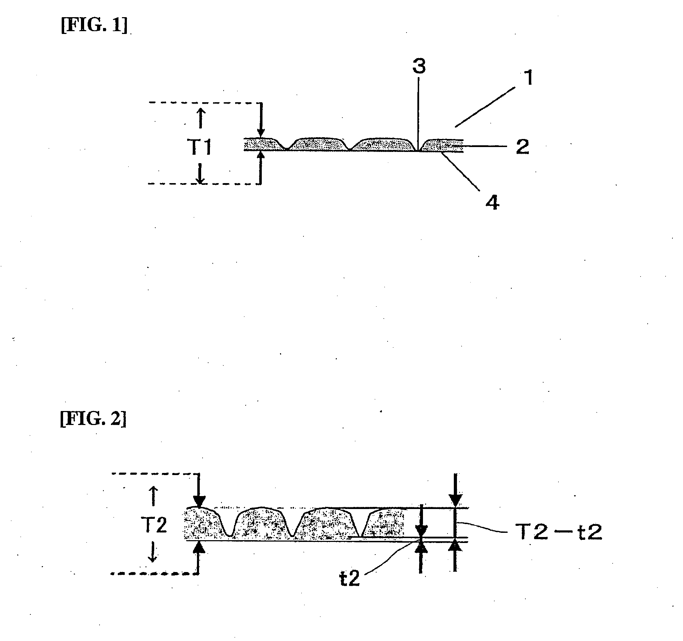

As to the sample obtained, a thickness of the plane not subjected to embossing was measured using a laser displacement sensor (manufactured by KEYENCE Corporation, model number: LB series). The measurements of the thickness were made five times with changing the measurement sites, and an average was defined as a thickness T1 (mm) before absorption of the saline solution (see, for example, Figure 1).

Ten milliliters of the saline solution (0.9% by mass aqueous sodium chloride solution, hereinafter referred to the same) was evenly supplied to the above-mentioned sample to be absorbed. Here, the absorbed saline solution corresponds to 4 L per 1 $m^2$ of sample (water-absorbent sheet structure) (4 $L/m^2$).

As to a sample after absorption of the saline solution at 10 minutes passed from supplying the saline solution, a thickness T2 (mm) of the sample after absorption of the saline solution was measured in accordance with the same measurement method as that of T1 (see, for example, Figure 2).

In addition, a thickness at a site subjected to embossing was measured with a laser displacement sensor (manufactured by KEYENCE Corporation, model number: LB series). The measurements of the embossing depth were made five times with changing the measurement sites, and an average was defined as an embossing thickness t2 (mm) after absorption of the saline solution (see, for example, Figure 2).

After the measurements of T1, T2 and t2 mentioned above, the expansion thickness ratio and the expansion embossing depth of the water-absorbent sheet structure were each calculated in accordance with the following formulas.

$$(A) \qquad \text{Expansion Thickness Ratio} = T2/T1$$

$$(B) \qquad \text{Expansion Embossing Depth} = (T2 - t2)/T2$$

[Permeation Rate, Liquid Leakage in Width Direction and Amount of Re-wet of Water-Absorbent Sheet Structure]

A water-absorbent sheet structure, which was cut into rectangular strips having dimensions of 10 cm × 30 cm in a manner that a longitudinal direction thereof is to be in a length direction (machine feeding direction) of the hydrophilic nonwoven fabric, was used as a sample.

In a 10 L container were placed 60 g of sodium chloride, 1.8 g of calcium chloride dihydrate, 3.6 g of magnesium chloride hexahydrate, and a proper amount of distilled water to completely dissolve. Next, 15 g of an aqueous 1% by mass poly(oxyethylene) isooctylphenyl ether solution was added thereto, and distilled water was further added to adjust the mass of the overall aqueous solution to 6,000 g. Thereafter, the mixed solution was colored with a small amount of Blue No. 1 to prepare a test solution.

A polyethylene air-through style porous liquid-permeable sheet having the same size as the sample (10 cm × 30 cm) and a basis weight of 22 $g/m^2$ was placed over an upper side of a sample (water-absorbent sheet structure). In addition, underneath the sample was placed a polyethylene liquid-impermeable sheet having the same size and basis weight as the sample, to prepare a simple absorbent article. A cylindrical cylinder having an inner diameter of 3 cm was placed near the central section of this absorbent article, and a 50 mL test solution was supplied into the cylinder at one time. At the same time, a time period until the test solution was completely lost from the cylinder was measured with a stopwatch, which is referred to as a first permeation rate (seconds). Next, the same procedures were carried out placing the cylindrical cylinder at the same position as the first permeation rate 30 minutes thereafter and 60 minutes thereafter, to measure second and third permeation rates (seconds). A total of the number of seconds for the first to third permeation rates was referred to as a total permeation rate.

In addition, after the termination of the measurements of each of the above-mentioned first to third permeation rates, the presence or absence of liquid leakage in a width direction of the water-absorbent sheet structure was visually confirmed. If liquid leakage in a width direction took place even once, the water absorbent sheet structure was evaluated as presence for the liquid leakage.

Further, after 120 minutes from the start of the supplying of the first test solution, the cylinder was removed, filter papers (about 80 sheets) of 10 cm each side, of which mass (Wa (g), about 70 g) was previously measured, were stacked near the test solution supplying position on the absorbent article, and a 5 kg weight of which bottom side has dimensions of 10 cm × 10 cm was placed thereon. After 5 minutes of applying a load, the mass (Wb (g)) of the filter papers was measured, and an increased mass was defined as the amount of liquid re-wet (g) as follows.

$$\text{Amount of Liquid Re-wet (g)} = Wb - Wa$$

[Slope Leakage Test]

A slope leakage test was conducted using an apparatus shown in Figure 8.

Schematically, a commercially available stand 51 for experimental facilities was used to slope an acrylic plate 52 and fixed, the above-mentioned test solution was then supplied to an absorbent article 53 placed on the plate from a dropping funnel 54 positioned vertically above the absorbent article, and a leakage amount was measured with a balance 55. The detailed specifications are given hereinbelow.

An acrylic plate 52 has a length in the direction of the slope plane of 45 cm, and fixed so that an angle formed with a stand 51 against the horizontal is 45° ± 2°. The acrylic plate 52 had a width of 100 cm and a thickness of 1 cm, and plural absorbent articles 53 could be concurrently measured. The acrylic plate 52 had a smooth surface, so that the test solution was not detained or absorbed to the plate.

A dropping funnel 54 was fixed at a position vertically above the sloped acrylic plate 52 using the stand 51. The dropping funnel 54 had a volume of 100 mL, and an inner diameter of a tip end portion of about 4 mm, and an aperture of the cock was adjusted so that a liquid was supplied at a rate of 8 mL/s.

A balance 55 on which a tray 56 was placed was set at a lower side of the acrylic plate 52, and all the test solutions flowing down the plate were received as leakage, and the mass was recorded to the accuracy of 0.1 g.

A slope leakage test using an apparatus as described above was carried out in accordance with the following procedures. The mass of a water-absorbent sheet structure cut into a rectangular strip having dimensions of width × length 10 cm × 30 cm in a manner that the longitudinal direction is a length direction (machine feeding direction) of the hydrophilic nonwoven fabric was measured. Next, an air through-style polyethylene liquid-permeable nonwoven fabric (basis weight: 22 g/m$^2$) of the same size was attached from an upper side thereof, and further a polyethylene liquid-impermeable sheet having the same size and the same basis weight was attached from a lower side thereof to prepare a simple absorbent article 53. The simple absorbent article 53 was adhered on the acrylic plate 52 (in order not to stop leakage intentionally, the bottom end of the absorbent article 53 was not adhered to the acrylic plate 52).

Marking was put on the absorbent article 53 at a position 2 cm away in a downward direction from a top end thereof, and a supplying inlet for the dropping funnel 54 was fixed so that the inlet was positioned at a distance 8 mm ± 2 mm vertically above the marking.

A balance 55 was turned on, and tared so that the indication was zero, and thereafter 80 mL of the above-mentioned test solution was supplied at one time to the dropping funnel 54. An amount of liquid poured into a tray 56 after the test solution was allowed to flow over a sloped acrylic plate 52 without being absorbed into an absorbent article 53 was measured, and this amount of liquid was defined as a first leakage amount (g). The numerical value for this first leakage amount (g) was denoted as LW1.

Second and third test solutions were supplied in 10-minute intervals from the beginning of the first supply, and second and third leakage amounts (g) were measured, and the numerical values therefor were respectively denoted as LW2 and LW3.

Next, a leakage index was calculated in accordance with the following equation. The smaller the index, the smaller the leakage amount at a slope of a water-absorbent sheet structure, especially an initial leakage amount, whereby it is judged to be an excellent water-absorbent sheet structure.

$$\text{Leakage Index:} \quad L = LW1 \times 10 + LW2 \times 5 + LW3$$

[Deformation in Form of Water-Absorbent Sheet Structure] Changes in states of the water-absorbent sheet structure after carrying out the slope leakage test were confirmed with eyes, and evaluated in accordance with the following criteria.

A: No changes took place in an absorbent layer, and the sheet structure did not undergo deformation in the form.
B: Change in an absorbent layer such as migration of a water-absorbent resin partially took place, and the sheet structure underwent some deformation in the form.
C: The sheet structure underwent considerable deformation in the form.

[Example 1]

A spunbond-meltblown-spunbond (hereinafter denoted as SMS) nonwoven fabric made of polypropylene having a width of 30 cm hydrophilically treated with a hydrophilic treatment agent (basis weight: 13 g/m$^2$, thickness: 150 μm, polypropylene content: 100%) was spread as a hydrophilic nonwoven fabric over a hot melt applicator (manufactured by HALLYS Corporation, Marshall 150) of which heating temperature was set at 150°C, and thereafter a styrene-butadiene-styrene copolymer (SBS-1; softening point: 85°C, holding strength: 1,440 minutes or longer) was coated as an adhesive over the nonwoven fabric at a basis weight of 14 g/m$^2$

Next, a roller spreader (manufactured by HASHIMA CO., LTD., SINTERACE M/C) was charged at its supplying inlet with a crosslinked product of a partially neutralized sodium salt of polyacrylic acid (manufactured by Sumitomo Seika Co., Ltd., AQUAKEEP SA55SX-II) as a water-absorbent resin. On the other hand, the above-mentioned adhesive-coated, hydrophilic nonwoven fabric was spread over a conveyor at the bottom side of the spreader. Subsequently, the spreading roller and the bottom side conveyor were operated, thereby allowing the above-mentioned crosslinked product of a partially neutralized sodium salt of polyacrylic acid to evenly overlay over the above-mentioned adhesive-coated, hydrophilic nonwoven fabric at a basis weight of 190 g/m$^2$, to give an overlaid product.

The overlaid product obtained was pressed from a top part with the above-mentioned SMS hydrophilic nonwoven fabric coated with the above-mentioned SBS-1 at a basis weight of 14 g/m$^2$ as an adhesive in the same manner as described above, and heat-fused with a laminating machine (manufactured by HASHIMA CO., LTD., straight linear fusing press HP-600LF) of which heating temperature was set at 100°C to integrate, to give an intermediate product of a water-absorbent sheet structure.

The intermediate product of a water-absorbent sheet structure obtained was spread over the hot melt applicator of which heating temperature was set at 150°C in the same manner as described above, and the above-mentioned SBS-1 was coated as an adhesive over the above-mentioned intermediate product of a water-absorbent sheet structure at a basis weight of 10 g/m$^2$.

Next, the above-mentioned roller spreader was charged at its supplying inlet with a crosslinked product of a partially neutralized sodium salt of polyacrylic acid (manufactured by Sumitomo Seika Co., Ltd., AQUAKEEP 10SH-PB) as a water-absorbent resin. On the other hand, the above-mentioned adhesive-coated intermediate product of a water-absorbent sheet structure was spread over a conveyor at the bottom part of the spreader. Next, the spreading roller and the bottom part conveyor were operated, thereby allowing the above-mentioned crosslinked product of a partially neutralized sodium salt of polyacrylic acid to evenly overlay over the adhesive-coated intermediate product of a water-absorbent sheet structure at a basis weight of 50 g/m$^2$, to give an overlaid product.

The overlaid product obtained was pressed from a top part with the above-mentioned SMS hydrophilic nonwoven fabric coated with the above-mentioned SBS-1 at a basis weight of 10 g/m$^2$ as an adhesive in the same manner as described above, and thereafter heat-fused with the above-mentioned laminating machine of which heating temperature was set at 100°C to integrate, to give a water-absorbent sheet structure before being subjected to embossing.

The water-absorbent sheet structure before being subjected to embossing obtained was cut into rectangular strips having dimensions of 10 cm × 30 cm in a manner that a longitudinal direction thereof is to be in a length direction (machine feeding direction) of the nonwoven fabric, and thereafter the embossing was formed on (one side of) the above-mentioned water-absorbent sheet structure with a thermal embossing roller in a manner that the embossing had an areal percentage of 7% and the embossing patterns as shown in Figure 7, to give a water-absorbent sheet structure.

The above-mentioned various measurements and evaluations were made for the resulting water-absorbent sheet structure. The results are shown in Tables 1 and 2.

[Example 2]

The same procedures as in Example 1 were carried out except that the embossing formed had an areal percentage changing from 7% to 13%, and the embossing patterns as shown in Figure 6, to give a water-absorbent sheet structure.

The above-mentioned various measurements and evaluations were made for the resulting water-absorbent sheet structure. The results are shown in Tables 1 and 2.

[Example 3]

A spunlace hydrophilic nonwoven fabric having a width of 30 cm (basis weight: 50 g/m$^2$, thickness: 400 μm, rayon content: 70%, polyethylene terephthalate content: 30%) was spread as a hydrophilic nonwoven fabric over a hot melt applicator (manufactured by HALLYS Corporation, Marshall 150) of which heating temperature was set at 150°C, and thereafter a styrene-butadiene-styrene copolymer (SBS-1; softening point: 85°C, holding strength: 1,440 minutes or longer) was coated as an adhesive over the nonwoven fabric at a basis weight of 20 g/m$^2$.

Next, a roller spreader (manufactured by HASHIMA CO., LTD., SINTERACE M/C) was charged at its supplying inlet with a crosslinked product of a partially neutralized sodium salt of polyacrylic acid (manufactured by Sumitomo Seika Co., Ltd., AQUAKEEP SA55SX-II) as a water-absorbent resin. On the other hand, the above-mentioned adhesive-coated hydrophilic nonwoven fabric was spread over a conveyor at the bottom side of the spreader. Subsequently, the spreading roller and the bottom side conveyor were operated, thereby allowing the above-mentioned crosslinked product of a partially neutralized sodium salt of polyacrylic acid to evenly overlay over the above-mentioned adhesive-coated

hydrophilic nonwoven fabric at a basis weight of 270 g/m$^2$, to give an overlaid product.

The overlaid product obtained was pressed from a top part with the above-mentioned spunlace nonwoven fabric coated with the above-mentioned SBS-1 at a basis weight of 20 g/m$^2$ as an adhesive in the same manner as described above, and heat-fused with a laminating machine (manufactured by HASHIMA CO., LTD., straight linear fusing press HP-600LF) of which heating temperature was set at 100°C to integrate, to give an intermediate product of a water-absorbent sheet structure A-1.

The spunlace hydrophilic nonwoven fabric (basis weight: 50 g/m$^2$, thickness: 400 μm, rayon content: 70%, polyethylene terephthalate content: 30%) having a width of 30 cm as a hydrophilic nonwoven fabric was spread over the above-mentioned hot melt applicator of which heating temperature was set at 150°C in the same manner as described above, and thereafter the SBS-1 was coated as an adhesive over the nonwoven fabric at a basis weight of 6 g/m$^2$.

Next, the above-mentioned roller spreader was charged at its supplying inlet with a crosslinked product of a partially neutralized sodium salt of polyacrylic acid (manufactured by Sumitomo Seika Co., Ltd., AQUAKEEP 10SH-PB) as a water-absorbent resin. On the other hand, the above-mentioned adhesive-coated, hydrophilic nonwoven fabric was spread over a conveyor at the bottom part of the spreader. Next, the spreading roller and the bottom part conveyor were operated, thereby allowing the above-mentioned crosslinked product of a partially neutralized sodium salt of polyacrylic acid to evenly overlay over the above-mentioned adhesive-coated, hydrophilic nonwoven fabric at a basis weight of 70 g/m$^2$, to give an overlaid product.

The overlaid product obtained was pressed from a top part with the above-mentioned spunlace hydrophilic nonwoven fabric coated with the above-mentioned SBS-1 at a basis weight of 6 g/m$^2$ as an adhesive in the same manner as described above, and heat-fused with the above-mentioned laminating machine of which heating temperature was set at 100°C to integrate, to give an intermediate product of a water-absorbent sheet structure B-1.

The above-mentioned SBS-1 was coated over the intermediate product of a water-absorbent sheet structure B-1 obtained at a basis weight of 4 g/m$^2$ in the same manner as described above, and thereafter the intermediate product of a water-absorbent sheet structure A-1 obtained was overlaid from the top thereof. Subsequently, the overlaid product was heat-fused with the above-mentioned laminating machine of which heating temperature was set at 40°C to integrate the intermediate products of water-absorbent sheet structures A-1 and B-1, to give a water-absorbent sheet structure before being subjected to embossing.

The water-absorbent sheet structure before being subjected to embossing obtained was cut into rectangular strips having dimensions of 10 cm × 30 cm in a manner that a longitudinal direction thereof is to be in a length direction (machine feeding direction) of the nonwoven fabric, and thereafter the embossing was formed on (one side of) the above-mentioned water-absorbent sheet structure with a thermal embossing roller in a manner that the embossing had an areal percentage of 7% and the embossing patterns as shown in Figure 7, to give a water-absorbent sheet structure.

The above-mentioned various measurements and evaluations were made for the resulting water-absorbent sheet structure. The results are shown in Tables 1 and 2.

[Example 4]

The intermediate product of a water-absorbent sheet structure A-1 before being subjected to embossing obtained in Example 3 was cut into rectangular strips having dimensions of 10 cm × 30 cm in a manner that a longitudinal direction thereof is to be in a length direction (machine feeding direction) of the nonwoven fabric, and thereafter the embossing was formed on (one side of) the above-mentioned intermediate product of a water-absorbent sheet structure A-1 with a thermal embossing roller in a manner that the embossing had an areal percentage of 7% and the embossing patterns as shown in Figure 7, to give an intermediate product of a water-absorbent sheet structure A-1e.

The intermediate product of a water-absorbent sheet structure B-1 obtained in Example 3 was cut into rectangular strips having dimensions of 10 cm × 30 cm in a manner that a longitudinal direction thereof is to be in a length direction (machine feeding direction) of the nonwoven fabric, and thereafter the above-mentioned SBS-1 was coated over the intermediate product of a water-absorbent sheet structure B-1 at a basis weight of 4g/m$^2$ in the same manner as described above. Thereafter, a side of the intermediate product of a water-absorbent sheet structure A-1e obtained not subjected to embossing was overlaid from a top thereof. Subsequently, the above-mentioned intermediate products of water-absorbent sheet structures A-1e and B-1 were heat-fused with the above-mentioned laminating machine of which heating temperature was set at 40°C to integrate, to give a water-absorbent sheet structure.

[Example 5]

A spunlace hydrophilic nonwoven fabric having a width of 30 cm (basis weight: 50 g/m$^2$, thickness: 400 μm, rayon content: 70%, polyethylene terephthalate content: 30%) was spread as a hydrophilic nonwoven fabric over a hot melt applicator (manufactured by HALLYS Corporation, Marshall 150) of which heating temperature was set at 150°C, and thereafter a styrene-butadiene-styrene copolymer (SBS-1; softening point: 85°C, holding strength: 1,440 minutes or longer) was coated as an adhesive over the nonwoven fabric at a basis weight of 30 g/m$^2$.

Next, a roller spreader (manufactured by HASHIMA CO., LTD., SINTERACE M/C) was charged at its supplying inlet with a crosslinked product of a partially neutralized sodium salt of polyacrylic acid (manufactured by Sumitomo Seika Co., Ltd., AQUAKEEP SA55SX-II) as a water-absorbent resin. On the other hand, the above-mentioned adhesive-coated, hydrophilic nonwoven fabric was spread over a conveyor at the bottom side of the spreader. Subsequently, the spreading roller and the bottom side conveyor were operated, thereby allowing the above-mentioned crosslinked product of a partially neutralized sodium salt of polyacrylic acid to evenly overlay over the above-mentioned adhesive-coated, hydrophilic nonwoven fabric at a basis weight of 400 g/m$^2$, to give an overlaid product.

The overlaid product obtained was pressed from a top part with the above-mentioned spunlace hydrophilic nonwoven fabric coated with the above-mentioned SBS-1 at a basis weight of 30 g/m$^2$ as an adhesive in the same manner as described above, and heat-fused with a laminating machine (manufactured by HASHIMA CO., LTD., straight linear fusing press HP-600LF) of which heating temperature was set at 100°C to integrate, to give a water-absorbent sheet structure before being subjected to embossing.

The resulting water-absorbent sheet structure before being subjected to embossing was cut into rectangular strips having dimensions of 10 cm × 30 cm in a manner that a longitudinal direction thereof is to be in a length direction (machine feeding direction) of the nonwoven fabric, and thereafter the embossing was formed on (one side of) the above-mentioned water-absorbent sheet structure with a thermal embossing roller in a manner that the embossing had an areal percentage of 7% and the embossing patterns as shown in Figure 7, to give a water-absorbent sheet structure.

The above-mentioned various measurements and evaluations were made for the resulting water-absorbent sheet structure. The results are shown in Tables 1 and 2.

[Comparative Example 1]

The same procedures as in Example 1 were carried out except that the overlaid sheet structure was not subjected to embossing, to give a water-absorbent sheet structure.

The above-mentioned various measurements and evaluations were made for the resulting water-absorbent sheet structure. The results are shown in Tables 1 and 2.

[Comparative Example 2]

The same procedures as in Example 1 were carried out except that the adhesive was changed from the styrene-butadiene-styrene copolymer (SBS-1; softening point: 85°C, holding strength: 1,440 minutes or longer) to styrene-isoprene-styrene copolymer (SBS-2; softening point: 82°C, holding strength: 850 minutes), to give a water-absorbent sheet structure.

The above-mentioned various measurements and evaluations were made for the resulting water-absorbent sheet structure. The results are shown in Tables 1 and 2.

[Comparative Example 3]

The same procedures as in Example 1 were carried out except that the embossing formed had an areal percentage changing from 7% to 35%, and the embossing patterns as shown in Figure 6, to give a water-absorbent sheet structure.

The above-mentioned various measurements and evaluations were made for the resulting water-absorbent sheet structure. The results are shown in Tables 1 and 2.

[Comparative Example 4]

A spunlace hydrophilic nonwoven fabric having a width of 30 cm (basis weight: 50 g/m$^2$, thickness: 400 $\mu$m, rayon content: 70%, polyethylene terephthalate content: 30%) was spread over a hot melt applicator (manufactured by HALLYS Corporation, Marshall 150) of which heating temperature was set at 150°C as a hydrophilic nonwoven fabric, and thereafter a styrene-butadiene-styrene copolymer (SBS-1; softening point: 85°C, holding strength: 1,440 minutes or longer) was coated as an adhesive over the nonwoven fabric at a basis weight of 16 g/m$^2$.

Next, a roller spreader (manufactured by HASHIMA CO., LTD., SINTERACE M/C) was charged at its supplying inlet with a crosslinked product of a partially neutralized sodium salt of polyacrylic acid (manufactured by Sumitomo Seika Co., Ltd., AQUAKEEP SA55SX-II) as a water-absorbent resin. On the other hand, the above-mentioned adhesive-coated, hydrophilic nonwoven fabric was spread over a conveyor at the bottom side of the spreader. Subsequently, the spreading roller and the bottom side conveyor were operated, thereby allowing the above-mentioned crosslinked product of a partially neutralized sodium salt of polyacrylic acid to evenly overlay over the above-mentioned adhesive-coated,

hydrophilic nonwoven fabric at a basis weight of 220 g/m², to give an overlaid product.

The overlaid product obtained was pressed from a top part with the above-mentioned spunlace hydrophilic nonwoven fabric coated with the above-mentioned SBS-1 at a basis weight of 16 g/m² as an adhesive in the same manner as described above, and heat-fused with a laminating machine (manufactured by HASHIMA CO., LTD., straight linear fusing press HP-600LF) of which heating temperature was set at 100°C to integrate, to give an intermediate product of a water-absorbent sheet structure A-2 before being subjected to embossing.

The intermediate product of a water-absorbent sheet structure A-2 before being subjected to embossing obtained was cut into rectangular strips having dimensions of 10 cm × 30 cm in a manner that a longitudinal direction thereof is to be in a length direction (machine feeding direction) of the nonwoven fabric, and thereafter the embossing was formed on (one side of) the above-mentioned intermediate product of a water-absorbent sheet structure A-2 with a thermal embossing roller in a manner that the embossing had an areal percentage of 7% and the embossing patterns as shown in Figure 7, to give an intermediate product of a water-absorbent sheet structure A-2e.

A spunlace hydrophilic nonwoven fabric having a width of 30 cm (basis weight: 50 g/m², thickness: 400 $\mu$m, rayon content: 70%, polyethylene terephthalate content: 30%) was spread over the above-mentioned hot melt applicator of which heating temperature was set at 150°C as a hydrophilic nonwoven fabric in the same manner as described above, and thereafter a styrene-isoprene-styrene copolymer (SBS-2; softening point: 82°C, holding strength: 850 minutes) was coated as an adhesive over the nonwoven fabric at a basis weight of 9 g/m².

Next, the above-mentioned roller spreader was charged at its supplying inlet with a crosslinked product of a partially neutralized sodium salt of polyacrylic acid (manufactured by Sumitomo Seika Co., Ltd., AQUAKEEP 10SH-PB) as a water-absorbent resin. On the other hand, the above-mentioned adhesive-coated, hydrophilic nonwoven fabric was spread over a conveyor at the bottom side of the spreader. Subsequently, the spreading roller and the bottom side conveyor were operated, thereby allowing the above-mentioned crosslinked product of a partially neutralized sodium salt of polyacrylic acid to evenly overlay over the above-mentioned adhesive-coated, hydrophilic nonwoven fabric at a basis weight of 120 g/m², to give an overlaid product.

The overlaid product obtained was pressed from a top part with the above-mentioned spunlace hydrophilic nonwoven fabric coated with the above-mentioned SBS-2 at a basis weight of 9 g/m² as an adhesive in the same manner as described above, and thereafter heat-fused with the above-mentioned laminating machine of which heating temperature was set at 100°C to integrate, to give an intermediate product of a water-absorbent sheet structure B-2.

The intermediate product of water-absorbent sheet structure B-2 obtained was cut into rectangular strips having dimensions of 10 cm × 30 cm in a manner that a longitudinal direction thereof is to be in a length direction (machine feeding direction) of the nonwoven fabric, and thereafter the above-mentioned SBS-1 was coated over the intermediate product of the water-absorbent sheet structure B-2 at a basis weight of 4 g/m² in the same manner as described above. Thereafter, a side not subjected to embossing of the intermediate product of the water-absorbent sheet structure A-2e obtained was overlaid from a top thereof. Subsequently, the above-mentioned intermediate products of the water-absorbent sheet structures A-2e and B-2 were heat-fused with the above-mentioned laminating machine of which heating temperature was set at 40°C to integrate, to give a water-absorbent sheet structure.

The above-mentioned various measurements and evaluations were made for the resulting water-absorbent sheet structure. The results are shown in Tables 1 and 2.

[Table 1]

Table 1

| | Areal Percentage of Embossing [%] | Embossing Patterns | Thickness [mm] | T1 [mm] | T2 [mm] | t2 [mm] | T2 - t2 [mm] | Expansion Thickness Ratio | Expansion Embossing Depth |
|---|---|---|---|---|---|---|---|---|---|
| Examples | | | | | | | | | |
| 1 | 7 | Figure 7 | 1.2 | 1.2 | 5.5 | 0.5 | 5.0 | 4.6 | 0.91 |
| 2 | 13 | Figure 6 | 1.1 | 1.2 | 5.3 | 0.3 | 5.0 | 4.4 | 0.94 |
| 3 | 7 | Figure 7 | 2.2 | 2.2 | 6.2 | 0.6 | 5.6 | 2.8 | 0.90 |
| 4 | 7 | Figure 7 | 2.2 | 2.2 | 6.4 | 1.6 | 4.8 | 2.9 | 0.75 |
| 5 | 7 | Figure 7 | 0.9 | 0.9 | 3.4 | 0.4 | 3.0 | 3.8 | 0.88 |
| Comparative Examples | | | | | | | | | |
| 1 | - | - | 1.2 | 1.2 | 6.2 | - | - | 5.2 | - |
| 2 | 7 | Figure 7 | 1.2 | 1.2 | 6.1 | 5.6 | 0.5 | 5.1 | 0.08 |
| 3 | 35 | Figure 6 | 1.1 | 1.1 | 2.0 | 0.3 | 1.7 | 1.8 | 0.85 |
| 4 | 7 | Figure 7 | 2.2 | 2.3 | 7.0 | 3.2 | 3.8 | 3.0 | 0.54 |

T1: a thickness (mm) of a water-absorbent sheet structure before absorption of the saline solution;
T2: a thickness (mm) of the water-absorbent sheet structure after absorption of the saline solution; and
t2: an embossing thickness (mm) in a water-absorbent sheet structure after absorption of the saline solution.

[Table]

Table 2

| | Permeation Rate [sec] | | | | Amount of Re-wet [g] | Liquid Leakage in Width Direction | Slope Leakage Test | | | | Deformation of Form |
|---|---|---|---|---|---|---|---|---|---|---|---|
| | 1 | 2 | 3 | Total | | | LW1 | LW2 | LW3 | Index | |
| Examples 1 | 18 | 9 | 9 | 36 | 9.7 | Absence | 0.0 | 0.0 | 0.0 | 0 | A |
| 2 | 17 | 8 | 7 | 32 | 9.9 | Absence | 0.0 | 3.1 | 4.7 | 20 | A |
| 3 | 19 | 10 | 9 | 38 | 1.7 | Absence | 0.0 | 1.1 | 3.3 | 9 | A |
| 4 | 22 | 11 | 10 | 43 | 1.8 | Absence | 0.0 | 0.0 | 2.1 | 2 | A |
| 5 | 18 | 10 | 12 | 40 | 1.2 | Absence | 2.6 | 1.2 | 0.8 | 33 | A |
| Comparative Examples 1 | 36 | 15 | 17 | 68 | 15.0 | Absence | 2.0 | 0.0 | 0.0 | 20 | A |
| 2 | 23 | 11 | 18 | 52 | 14.4 | Absence | 0.0 | 0.0 | 0.0 | 0 | B |
| 3 | 15 | 8 | 6 | 29 | 9.8 | Presence | 8.2 | 26.7 | 35.6 | 251 | A |
| 4 | 24 | 13 | 15 | 52 | 1.6 | Absence | 0.0 | 0.0 | 0.0 | 0 | B |

It could be seen from the above results that the water-absorbent sheet structures of Examples had a fast liquid permeation rate and a small amount of liquid leakage in a width direction, and did not undergo deformation of the form after absorption of liquid (excellent shape retaining ability).

On the other hand, in Comparative Examples, all of a water-absorbent sheet structure not subjected to embossing (Comparative Example 1), water-absorbent sheet structures of which embossing depth would be shallow when the sheet structures absorb a liquid to allow swelling (Comparative Examples 2 and 4), and a sheet structure of which expansion thickness ratio is small (Comparative Example 3) cannot simultaneously satisfy the objectives such as improvement in liquid permeation rate, prevention of liquid leakage in a width direction, and deformation of the form, so that they are inferior as water-absorbent sheet structures.

INDUSTRIAL APPLICABILITY

The water-absorbent sheet structure of the present invention has an excellent liquid permeability, a small amount of liquid leakage and an excellent shape retaining ability, and accomplishes thinning, so that the water-absorbent sheet structure of the present invention can be suitably used for absorbent articles as represented by disposable diapers and the like.

EXPLANATION OF NUMERICAL SYMBOLS

| 1 | water-absorbent sheet structure |
|---|---|
| 2 | absorbent layer |
| 3 | embossing |
| 4 | hydrophilic nonwoven fabric |
| 51 | stand |
| 52 | acrylic plate |
| 53 | absorbent article |
| 54 | dropping funnel |
| 55 | balance |
| 56 | tray |

**Claims**

1. A water-absorbent sheet structure (1) comprising a structure in which an absorbent layer (2) containing a water-absorbent resin is sandwiched with a hydrophilic nonwoven fabric (4) from an upper side and a lower side of the absorbent layer, **characterized in that** at least one side of a topside and an underside of the water-absorbent sheet structure is subjected to embossing (3), with an areal proportion of from 3 to 25%, based on the area of the side subjected to embossing of the water-absorbent sheet structure, and that the water-absorbent sheet structure has the following properties:

   when a saline solution is allowed to be absorbed in an amount of 4 L per 1 $m^2$ of the water-absorbent sheet structure (4 $L/m^2$), the water-absorbent sheet structure satisfies both of the following relationships of (A) and (B):

   (A) an expansion thickness ratio, i.e. T2/T1, of 2 or more, and
   (B) an expansion embossing depth, i.e. (T2 - t2) / T2, of 0.7 or more,

   wherein T1 is a thickness (mm) of the water-absorbent sheet structure before absorption of the saline solution; T2 is a thickness (mm) of the water-absorbent sheet structure after absorption of the saline solution; and t2 is an embossing thickness (mm) in the water-absorbent sheet structure after absorption of the saline solution, and
   wherein T1, T2 and t2 are thicknesses as measured using instrument model number: LB series, manufactured by KEYENCE Corporation.

2. The water-absorbent sheet structure according to claim 1,
   wherein the water-absorbent sheet structure has a thickness of 4 mm or less, in a dry state.

3. An absorbent article comprising the water-absorbent sheet structure as defined in anyone of claims 1 to 2, sandwiched between a liquid-permeable sheet and a liquid-impermeable sheet.

**Patentansprüche**

1. Wasserabsorbierende Folienstruktur (1), umfassend eine Struktur, in welcher eine absorbierende Schicht (2), enthaltend ein wasserabsorbierendes Harz, zwischen einem hydrophilen Vlies (4) von einer oberen Seite und einer unteren Seite der absorbierenden Schicht eingeschlossen ist, **dadurch gekennzeichnet, dass** mindestens eine Seite einer Oberseite und einer Unterseite der wasserabsorbierenden Folienstruktur einer Prägung (3) mit einem Flächenanteil von 3 bis 25% ausgesetzt ist, basierend auf der Fläche der Seite der wasserabsorbierenden Folienstruktur, die der Prägung ausgesetzt ist, und dass die wasserabsorbierende Folienstruktur die nachstehenden Eigenschaften aufweist:

   wenn eine Salzlösung in einer Menge von 4 L pro 1 $m^2$ der wasserabsorbierenden Struktur (4 $L/m^2$) absorbiert wird, erfüllt die wasserabsorbierende Folienstruktur beide der nachstehenden Verhältnisse von (A) und (B):

   (A) ein Expansionsdickeverhältnis, d.h. T2/T1, von 2 oder mehr, und
   (B) eine Expansionsprägetiefe, d.h. (T2 - t2) / T2 von 0,7 oder mehr,

   wobei T1 eine Dicke (mm) der wasserabsorbierenden Folienstruktur vor Absorption der Salzlösung ist; T2 eine Dicke (mm) der wasserabsorbierenden Folienstruktur nach Absorption der Salzlösung ist; und t2 eine Prägedicke (mm) in der wasserabsorbierenden Folienstruktur nach Absorption der Salzlösung ist, und
   wobei T1, T2 und t2 Dicken sind, die unter Verwendung von Geräteartikelnummer: LB series, hergestellt von der KEYENCE Corporation, gemessen sind.

2. Wasserabsorbierende Folienstruktur nach Anspruch 1, wobei die wasserabsorbierende Folienstruktur eine Dicke von 4 mm oder weniger in einem trockenen Zustand aufweist.

3. Absorbierender Artikel, umfassend die wasserabsorbierende Folienstruktur nach Anspruch 1 oder 2, eingeschlossen zwischen einer flüssigkeitsdurchlässigen Folie und einer flüssigkeitsundurchlässigen Folie.

**Revendications**

1. Structure en feuilles absorbant l'eau (1) comprenant une structure dans laquelle une couche absorbante (2) contenant une résine absorbant l'eau est insérée entre un tissu non tissé hydrophile (4) sur une face supérieure et une face inférieure de la couche absorbante, **caractérisée en ce que** la face supérieure et/ou la face inférieure de la structure en feuilles absorbant l'eau est soumise à un gaufrage (3), avec une proportion superficielle de 3 à 25 %, par rapport à la surface de la face soumise au gaufrage de la structure en feuilles absorbant l'eau, et **en ce que** la structure en feuilles absorbant l'eau possède les propriétés suivantes :

   lorsqu'on laisse une solution saline être absorbée en une quantité de 4 l par 1 $m^2$ de la structure en feuilles absorbant l'eau (4 $l/m^2$), la structure en feuilles absorbant l'eau satisfait aux deux relations (A) et (B) suivantes :

   (A) un rapport d'épaisseur de dilatation, à savoir T2/T1, de 2 ou plus, et
   (B) une profondeur de gaufrage de dilatation, à savoir (T2-t2)/T2, de 0,7 ou plus,

   dans lesquelles T1 est une épaisseur (mm) de la structure en feuilles absorbant l'eau avant absorption de la solution saline ; T2 est une épaisseur (mm) de la structure en feuilles absorbant l'eau après absorption de la solution saline ; et t2 est l'épaisseur de gaufrage (mm) dans la structure en feuilles absorbant l'eau après absorption de la solution saline, et
   dans lesquelles T1, T2 et t2 sont des épaisseurs mesurées à l'aide d'un numéro de modèle d'instrument : série LB, fabriqué par KEYENCE Corporation.

2. Structure en feuilles absorbant l'eau selon la revendication 1, dans laquelle la structure en feuilles absorbant l'eau a une épaisseur de 4 mm ou moins, à l'état sec.

3. Article absorbant comprenant la structure en feuilles absorbant l'eau selon l'une quelconque des revendications 1 à 2, insérée entre une feuille perméable aux liquides et une feuille imperméable aux liquides.

[FIG. 1]

[FIG. 2]

[FIG. 3]

[FIG. 4]

[FIG. 5]

[FIG. 6]

[FIG. 7]

[FIG. 8]

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2010004894 A
- JP HEI5300922 B
- US 20030028165 A